# EUROPÄISCHE PATENTSCHRIFT

(11) **EP 4 040 241 B1**
(45) Veröffentlichungstag und Bekanntmachung des Hinweises auf die Patenterteilung: **20.08.2025**
(21) Anmeldenummer: 21155033.0
(22) Anmeldetag: 03.02.2021
(51) Int. Cl.: G05B 19/042, A61C 7/04, A61C 19/04

(54) **MULTISENSORSYSTEM FÜR DEN INTRAORALBEREICH**
MULTISENSOR SYSTEM FOR INTRAORAL AREA
SYSTÈME DE CAPTEURS MULTIPLES POUR LA ZONE DE LA BOUCHE

(43) Veröffentlichungstag der Anmeldung: 10.08.2022
(73) Patentinhaber: Ivoclar Vivadent AG, 9494 Schaan (LI)
(72) Erfinder: Krybus, Robert Wolfgang, 9479 Oberschan (CH); Moosmann, Florian, 6835 Muntlix (AT)
(74) Vertreter: Baldus, Oliver

(56) Entgegenhaltungen:
- WO-A1-2013/121316
- WO-A1-2016/075511
- US-A1- 2015 064 640

## Beschreibung

Die vorliegende Erfindung betrifft ein Multisensorsystem mit mehreren Dentalsensoren zur Anordnung in einer Mundhöhle und ein Verfahren zum Übermitteln von Messdaten in einem Multisensorsystem.

Bei herkömmlichen Dentalsensoren liegt das Problem vor, dass nur ein gemeinsamer Übertragungskanal zum Empfänger zur Verfügung steht. Sobald mehrere Dentalsensoren gleichzeitig senden, kann es zu einer Kollision der Daten kommen. In diesem Fall können diese nicht ausgewertet werden.

WO2016/075511 zeigt ein intraorales multifunktionales Vibrationsgerät und ein drahtloses Gerät und zahnärztliches Patienteninteraktionssystem, das sich unter anderem dadurch auszeichnet, dass das besagte Gerät ein Empfänger für Sensoren und Mikrovibratoren ist, die von einer speziellen Hardware und Software betrieben werden.

Es ist die technische Aufgabe der vorliegenden Erfindung, ein Multisensorsystem mit mehreren Dentalsensoren bereitzustellen, mit dem Messdaten effizient übermittelt werden können.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Multisensorsystem gemäß Patentanspruch 1 gelöst.

Durch das Multisensorsystem kann eine effiziente, schnelle und störungsfreie Datenübertragung von einer Mehrzahl von Slave-Dentalsensoren erreicht werden. Die Messdaten aller Slave-Dentalsensoren werden an dem Master-Dentalsensor empfangen und können von diesem aus gebündelt an einen externen Knoten weitergeleitet werden.

In einer technisch vorteilhaften Ausführungsform des Multisensorsystems umfasst der Slave-Dentalsensor eine Befestigungsvorrichtung oder Befestigungsmittel zum Befestigen des Slave-Dentalsensors an einem Zahn und/oder der Master-Dentalsensor umfasst eine Befestigungsvorrichtung oder Befestigungsmittel zum Befestigen des Master-Dentalsensors an einem Zahn. Dadurch wird beispielsweise der technische Vorteil erreicht, dass das Multisensorsystem im Mund eines Patienten befestigt werden kann.

In einer weiteren technisch vorteilhaften Ausführungsform des Multisensorsystems umfasst die Sendevorrichtung des Slave-Dentalsensors eine drahtgebundene und/oder eine drahtlose Schnittstelle. Dadurch wird beispielsweise der technische Vorteil erreicht, dass die Messdaten schnell und einfach an den Master-Dentalsensor übertragen werden können.

In einer weiteren technisch vorteilhaften Ausführungsform des Multisensorsystems umfasst der Slave-Dentalsensor eine Empfangsvorrichtung zum Empfangen von Daten von dem Master-Dentalsensor. Dadurch wird beispielsweise der technische Vorteil erreicht, dass zusätzlich Daten von dem Master-Dentalsensor an den Slave-Dentalsensor übertragen werden können.

In einer weiteren technisch vorteilhaften Ausführungsform des Multisensorsystems ist der Slave-Dentalsensor ausgebildet, von dem Master-Dentalsensor gesteuert zu werden. Dadurch wird beispielsweise der technische Vorteil erreicht, dass Messungen auf Anforderung des Master-Dentalsensors ausgeführt werden können.

In einer weiteren technisch vorteilhaften Ausführungsform des Multisensorsystems umfasst der Master-Dentalsensor eine Sendevorrichtung zum Senden der Messdaten an einen Knoten außerhalb der Mundhöhle. Dadurch wird beispielsweise der technische Vorteil erreicht, dass die Messdaten gemeinsam auf einem Datenkanal an den externen Knoten zur weiteren Auswertung und Verarbeitung übertragen werden können.

In einer weiteren technisch vorteilhaften Ausführungsform des Multisensorsystems umfassen der Slave-Dentalsensor und/oder der Master-Dentalsensor einen Energiespeicher für einen autonomen Betrieb. Dadurch wird beispielsweise der technische Vorteil erreicht, dass der Slave-Dentalsensor oder der Master-Dentalsensor ohne externe Energieversorgungseinrichtung bereitgestellt werden können.

In einer weiteren technisch vorteilhaften Ausführungsform des Multisensorsystems umfasst der Master-Dentalsensor einen Mikroprozessor zum Verarbeiten der Messdaten. Dadurch wird beispielsweise der technische Vorteil erreicht, dass eine zentrale Verarbeitung der Messdaten erfolgen kann.

In einer weiteren technisch vorteilhaften Ausführungsform des Multisensorsystems ist der Master-Dentalsensor ausgebildet, die Messdaten zu komprimieren und/oder zu verschlüsseln. Dadurch wird beispielsweise der technische Vorteil erreicht, dass die Datenmenge reduziert wird oder eine sichere Kommunikation durchgeführt wird.

In einer weiteren technisch vorteilhaften Ausführungsform des Multisensorsystems umfasst der Slave-Dentalsensor einen digitalen Speicher zum Speichern eines Messprogramms und einen Mikroprozessor zum Ausführen des Messprogramms. Dadurch wird beispielsweise der technische Vorteil erreicht, dass der Slave-Dentalsensor flexibel durch ein Computerprogramm gesteuert werden kann.

In einer weiteren technisch vorteilhaften Ausführungsform des Multisensorsystems ist der Master-Dentalsensor ausgebildet, das Messprogramm auf dem Slave-Dentalsensor zu aktualisieren. Dadurch wird beispielsweise der technische Vorteil erreicht, dass nachträglich bessere und fehlerkorrigierte Versionen des Computerprogramms an den Slave-Dentalsensor übertragen werden können.

In einer weiteren technisch vorteilhaften Ausführungsform des Multisensorsystems ist das Multisensorsystem ausgebildet, die Messdaten an eine externe Applikation zu übermitteln. Die Applikation ist beispielsweise ein Computerprogramm auf einem Smart-Phone oder einem Tablet-PC. Die Übermittlung erfolgt beispielsweise über eine WLAN- oder eine Bluetooth-, oder NFC-Schnittstelle. Dadurch wird beispielsweise der technische Vorteil erreicht, dass die Messdaten auf einfache Weise auf einem tragbaren Gerät visualisiert werden können.

In einer weiteren technisch vorteilhaften Ausführungsform des Multisensorsystems ist der Slave-Dentalsensor ausgebildet, einen pH-Wert, eine Ethanol-Konzentration, eine Lactat-Konzentration, eine Cortisol-Konzentration, eine Glucose-Konzentration und/oder eine Ionenkonzentration zu erfassen oder Schallwellen beim Aufeinanderbeißen und/oder eine Temperatur zu erfassen. Dadurch wird beispielsweise der technische Vorteil erreicht, dass besonders geeignete Messdaten aus der Mundhöhle von den Slave-Dentalsensor erfasst werden können.

Gemäß einem zweiten Aspekt wird die technische Aufgabe durch eine Verwendung eines Multisensorsystem nach dem ersten Aspekt zum Erfassen von Messdaten in einer Mundhöhle gemäß Patentanspruch 13 gelöst.

Ausführungsbeispiele der Erfindung sind in den Zeichnungen dargestellt und werden im Folgenden näher beschrieben.

Es zeigen:
- Fig. 1: eine schematische Ansicht eines Multisensorsystem mit mehreren Dentalsensoren;
- Fig. 2: eine weitere schematische Ansicht des Multisensorsystems; und
- Fig. 3: ein Blockdiagramm eines Verfahrens zum Übermitteln von Messdaten in einem Multisensorsystem.

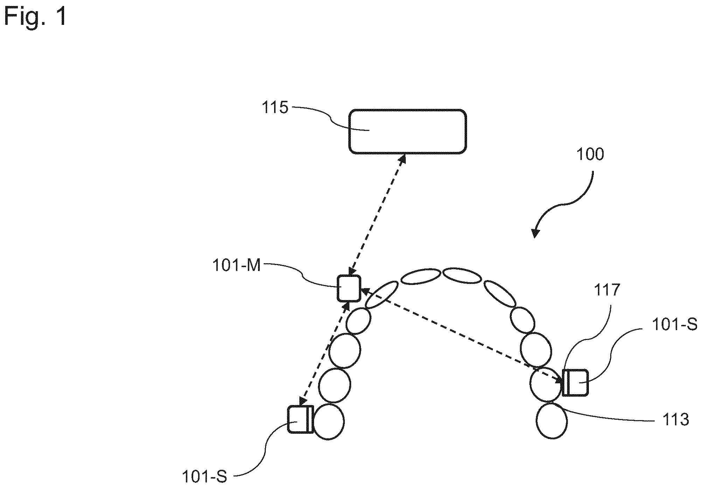

Fig. 1 zeigt eine schematische Ansicht eines Multisensorsystem 100 mit mehreren Dentalsensoren 101-S und 101-M. Die Dentalsensoren 101-S sind Slave-Dentalsensoren und der Dentalsensor 101-M ist ein Master-Dentalsensor. Die Slave-Dentalsensoren 101-S und/oder der Master-Dentalsensor 101-M können an einem Zahn 113 befestigt werden, beispielsweise mittels einer Befestigungsvorrichtung 117 oder jeglichen Befestigungsmittel, wie einem Kleber oder Zementen befestigt sein und unterschiedliche physikalische Größen oder Messwerte erfassen.

Die Slave-Dentalsensoren 101-S sind beispielsweise geeignet, um einen pH-Wert, eine Ethanol-Konzentration, eine Lactat-Konzentration, eine Cortisol-Konzentration, eine Glucose-Konzentration und/oder eine Ionenkonzentration als digitale Messdaten zu erfassen oder Schallwellen beim Aufeinanderbeißen und/oder eine Temperatur als digitale Messdaten zu erfassen. Im Allgemeinen können von den Slave-Dentalsensoren 101-S jedoch auch andere Größen bestimmt werden. Zu diesem Zweck weist jeder der Slave-Dentalsensoren 101-S eine geeignete Schaltung und Sensorik auf. Die Elektronik der Schaltung erfasst und verarbeitet die Messdaten auf geeignete Art und Weise.

Die Slave-Dentalsensoren 101-S übermitteln die erfassten Messdaten an den übergeordneten Master-Dentalsensor 101-M. Der Master-Dentalsensor 101-M ist den Slave-Dentalsensoren 101-S logisch zugeordnet und hierarchisch bei der Datenkommunikation übergeordnet. Der Master-Dentalsensor 101-M beherrscht die Zugriffsverhältnisse. Um den Master-Dentalsensor 101-M herum wird das Multisensorsystem 100 aufgebaut. Der Master-Dentalsensor 101-M empfängt die Messdaten von den jeweiligen Slave-Dentalsensoren 101-S. Der Master-Dentalsensor 101-M hat hierzu beispielsweise als einziger der Dentalsensoren 101 das Recht, unaufgefordert auf einen gemeinsamen Kommunikationskanal zuzugreifen. Auf diese Weise kann der Master-Dentalsensor 101-M die Messdaten nacheinander von den einzelnen Slave-Dentalsensoren 101-S abfragen. Auf diese Weise können Datenkollisionen verhindert werden, die dann entstehen, wenn mehrere Slave-Dentalsensoren 101-S gleichzeitig versuchen Messdaten zu senden.

Der Master-Dentalsensor 101-M ist in Datenkommunikation mit einem externen Knoten 115, an den die gesammelten Messdaten von den Slave-Dentalsensoren 101-S weitergeleitet werden. An diesem Knoten 115 findet beispielsweise eine Verarbeitung und eine Anzeige für einen Benutzer statt. Dieser Knoten ist beispielsweise durch einen Computer gebildet.

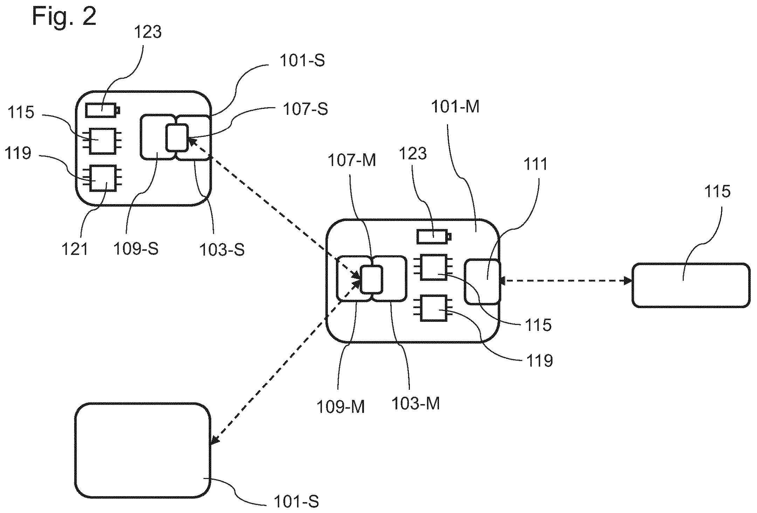

Fig. 2 zeigt eine weitere schematische Ansicht des Multisensorsystems 100. Die Sendevorrichtung 103-S des Slave-Dentalsensors 101-S umfasst eine drahtgebundene und/oder eine drahtlose Schnittstelle 107-S, die mit einer entsprechenden Schnittstelle 107-M einer Empfangsvorrichtung 109-M des Master-Dentalsensors 101-M kommuniziert. Die Schnittstellen 107-S und 107-M kommunizieren beispielsweise drahtlos über Funk, Bluetooth oder WLAN-Protokolle oder über Kabel miteinander in bidirektionaler Richtung. Auf diesem Weg können die Messdaten von dem Slave-Dentalsensor 101-S in der einen Richtung an den Master-Dentalsensor 101-M übertragen werden. In der anderen Richtung können Steuerdaten oder Messprogramme von dem Master-Dentalsensor 101-M an den Slave-Dentalsensor 101-S übertragen werden. Die Schnittstellen 107-S und Schnittstelle 107-M können die Daten bidirektional übermitteln, d.h. senden und empfangen. Zu diesem Zweck kann ein geeignetes Übertragungs- und Steuerungsprotokoll verwendet werden.

Dazu umfasst der Slave-Dentalsensors 101-S eine Sende-/Empfangsvorrichtung 109-S zum Empfangen von Daten von der Sende-/Empfangsvorrichtung 103-M des Master-Dentalsensors 101-M. Über die Empfangsvorrichtung 109-S kann der Slave-Dentalsensor 101-S beispielsweise Steuerdaten von dem Master-Dentalsensor 101-M empfangen. Die Steuerdaten können den Slave-Dentalsensor 101-S beispielsweise dazu veranlassen, eine vorgesehene Messung durchzuführen und die hierbei erfassten Messdaten an den Master-Dentalsensor 101-M zurückzusenden. Zu diesem Zweck wird eine Nachricht von dem Master-Dentalsensor 101-M an den Slave-Dentalsensor 101-S gesendet, die diesen zur Messung veranlasst.

Der Master-Dentalsensor 101-M umfasst eine weitere Sende-/Empfangsvorrichtung 111 zum Senden der Messdaten an einen externen Knoten 115 außerhalb der Mundhöhle und Empfangen von Daten. Der Knoten 115, der beispielsweise durch einen PC gebildet ist, kann die übermittelten Messdaten weiterverarbeiten und einem Benutzer anzeigen.

Sowohl der Slave-Dentalsensor 101-S als auch der Master-Dentalsensor 101-M umfassen einen elektrischen Energiespeicher 123 für einen autonomen Betrieb. Der Energiespeicher 123 versorgt die elektrischen Schaltungen des Slave-Dentalsensors 101-S oder des Master-Dentalsensors 101-M mit elektrischer Energie. Der Energiespeicher 123 ist beispielsweise eine Batterie oder ein Akkumulator.

Sowohl der Slave-Dentalsensor 101-S als auch der Master-Dentalsensor 101-M umfassen einen Mikroprozessor 115 zum Verarbeiten der Messdaten oder zum Ausführen von unterschiedlichen Computerprogrammen oder Betriebssystemen. Mithilfe dieses Mikroprozessors 115 können die Messdaten komprimiert/dekomprimiert und/oder entschlüsselt/verschlüsselt werden. Zusätzlich zum Mikroprozessor 115 ist ein digitaler Speicher 119 vorgesehen, um die ausführbare Messprogramme oder die Messdaten dauerhaft zu speichern. Der Master-Dentalsensor 101-M kann beispielsweise das Messprogramm 121 auf dem Slave-Dentalsensor 101-S aktualisieren, indem dieses von dem Master-Dentalsensor 101-M auf den Slave-Dentalsensor 101-S übertragen wird.

Alternativ kann der Benutzer mittels des Knotens 115 einen Sensor bestimmen, der als Master-Dentalsensor 101-M arbeiten soll. Zusätzlich ist es auch möglich, dass ein Multimastersystem zur Anwendung kommt, bei dem mehrere Master-Dentalsensoren bestimmt werden und zu jedem Mastersensor ein oder mehrere Slave-Dentalsensoren zugeordnet werden. Die mehreren Master-Dentalsensoren können vom Benutzer priorisiert werden, um eine Datenkollision zu verhindern.

Zur Verschlüsselung der Daten eignen sich verschiedene Kommunikationsprotokolle, wie beispielsweise eine Manchester-Codierung. Die einzelnen Slave- oder Master-Dentalsensoren 101-S und 101-M werden mittels der Verschlüsselung eindeutig adressiert, so dass jeder Sensor eindeutig identifiziert werden kann. Der Benutzer kann mittels des Knotens 115 frei auswählen, welche Daten wie oft und von welchem Sensor ausgelesen werden. Mit einer geeigneten Applikation (App) ist es möglich, dass die App die verschiedenen Messungen auswertet, Warnungen ausgibt oder Behandlungsmöglichkeiten vorschlägt.

Die App kann die Daten an eine Cloud übermitteln, zu der ein bestimmter Personenkreis Zugriff hat, wie z. B. Zahnärzte oder Zahntechniker. Der Personenkreis kann auf diese Daten und zugreifen und ebenfalls Daten auf dieser Cloud speichern, insbesondere Behandlungsempfehlungen abgeben, die dann der Benutzer in der App oder in der Cloud einsehen kann.

Im Weiteren kann die App diese Daten für anonyme statistische Auswertungen aufbereiten. Dadurch kann der Benutzer einsehen und seine Zähne mit den Zähnen anderer Benutzer der App vergleichen. Die App kann auch mittels künstlicher Intelligenz in der Lage sein, aus den Daten von verschiedenen Benutzern, eigenständig Behandlungsempfehlungen abzugeben oder Krankheiten festzustellen.

Die App kann auch Messungen von bestimmten Sensoren beauftragen, so dass beispielsweise am Morgen zunächst im Bereich der Backenzähne auf einer Seite, mittags im Bereich der Frontzähne und am Abend im Bereich der anderen Seite der Backenzähne Messungen durchgeführt werden. Möglich ist es auch, dass einen Tag lang nur immer an der gleichen Stelle Messungen durchgeführt werden.

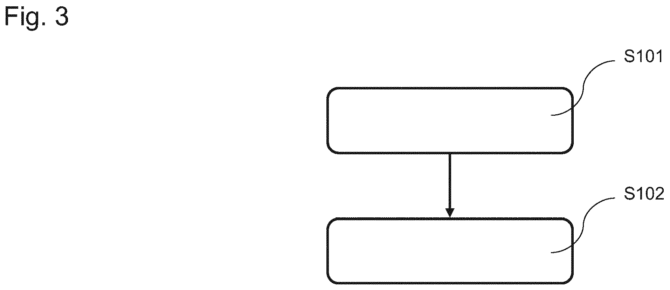

Fig. 3 zeigt ein Blockdiagramm eines Verfahrens zum Übermitteln von Messdaten in einem Multisensorsystem 100. Im Schritt S101 die Messdaten in der Mundhöhle durch einen Slave-Dentalsensor 101-S erfasst. Anschließend werden die Messdaten im Schritt S102 von dem Slave-Dentalsensoren 101-S an den Master-Dentalsensor 101-M gesendet. Auf diese Weise kann der Master-Dentalsensor 101-M die Messdaten von allen zugeordneten Slave-Dentalsensoren 101-S sammeln. Hierzu fragt der Master-Dentalsensor 101-M die Slave-Dentalsensoren 101-S beispielsweise mittels einer adressierten Nachricht nacheinander ab. Der Master-Dentalsensor 101-M kann ausgebildet sein, die Slave-Dentalsensoren 101-S kommunikationstechnisch zu steuern. Anschließend leitet der Master-Dentalsensor 101-M die gesammelten Messdaten an den externen Knoten 115 weiter. Der externe Knoten 115 kann durch die App auf einem Smartphone oder Tablet-PC gebildet sein.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Einrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Multisensorsystem
- 101-S: Slave-Dentalsensor
- 101-M: Master-Dentalsensor
- 103-S: Sendevorrichtung
- 103-M: Sendevorrichtung
- 107-S: Schnittstelle
- 107-M: Schnittstelle
- 109-S: Empfangsvorrichtung
- 109-M: Empfangsvorrichtung
- 111: Sendevorrichtung
- 113: Zahn
- 115: Knoten
- 117: Befestigungsvorrichtung
- 119: digitaler Speicher
- 121: Messprogramm
- 123: Energiespeicher

## Patentansprüche

1. Multisensorsystem (100) mit mehreren Dentalsensoren (101-S; 101-M) zur Anordnung in einer Mundhöhle, mit:
- mehreren Slave-Dentalsensoren (101-S) zum Erfassen von Messdaten in der Mundhöhle, die jeweils eine Sendevorrichtung (103) zum Senden der Messdaten umfassen; und
- einem Master-Dentalsensor (101-M) mit einer Empfangsvorrichtung (109-M) zum Empfangen der Messdaten von den einzelnen Slave-Dentalsensoren (101-S), der ausgebildet ist, Messdaten nacheinander von den einzelnen Slave-Dentalsensoren (101-S) abzufragen.

2. Multisensorsystem (100) nach Anspruch 1, wobei der Slave-Dentalsensor (101-S) eine Befestigungsvorrichtung (117) oder Befestigungsmittel zum Befestigen des Slave-Dentalsensors (101-S) an einem Zahn umfasst und/oder der Master-Dentalsensor (101-M) eine Befestigungsvorrichtung (117) oder Befestigungsmittel zum Befestigen des Master-Dentalsensors (101-M) an einem Zahn umfasst.

3. Multisensorsystem (100) nach einem der vorangehenden Ansprüche, wobei die Sendevorrichtung (103) des Slave-Dentalsensors (101-S) eine drahtgebundene und/oder eine drahtlose Schnittstelle (107) umfasst.

4. Multisensorsystem (100) nach einem der vorangehenden Ansprüche, wobei der Slave-Dentalsensor (101-S) eine Empfangsvorrichtung (109) zum Empfangen von Daten von dem Master-Dentalsensor (101-M) umfasst.

5. Multisensorsystem (100) nach einem der vorangehenden Ansprüche, wobei der Slave-Dentalsensor (101-S) ausgebildet ist, von dem Master-Dentalsensor (101-M) gesteuert zu werden.

6. Multisensorsystem (100) nach einem der vorangehenden Ansprüche, wobei der Master-Dentalsensor (101-M) eine Sendevorrichtung (111) zum Senden der Messdaten an einen Knoten (115) außerhalb der Mundhöhle umfasst.

7. Multisensorsystem (100) nach einem der vorangehenden Ansprüche, wobei der Slave-Dentalsensor (101-S) und/oder der Master-Dentalsensor (101-M) einen Energiespeicher für einen autonomen Betrieb umfassen.

8. Multisensorsystem (100) nach einem der vorangehenden Ansprüche, wobei der Master-Dentalsensor (101-M) einen Mikroprozessor (115) zum Verarbeiten der Messdaten umfasst.

9. Multisensorsystem (100) nach einem der vorangehenden Ansprüche, wobei der Master-Dentalsensor (101-M) ausgebildet ist, die Messdaten zu komprimieren und/oder zu verschlüsseln.

10. Multisensorsystem (100) nach einem der vorangehenden Ansprüche, wobei der Slave-Dentalsensor (101-S) einen digitalen Speicher (119) zum Speichern eines Messprogramms (121) und einen Mikroprozessor (115) zum Ausführen des Messprogramms (121) umfasst.

11. Multisensorsystem (100) nach einem der vorangehenden Ansprüche, wobei das Multisensorsystem (100) ausgebildet ist, die Messdaten an eine externe Applikation zu übermitteln.

12. Multisensorsystem (100) nach einem der vorangehenden Ansprüche, wobei der Slave-Dentalsensor (101-S) ausgebildet ist, einen pH-Wert, eine Ethanol-Konzentration, eine Lactat-Konzentration, eine Cortisol-Konzentration, eine Glucose-Konzentration und/oder eine Ionenkonzentration zu erfassen oder Schallwellen beim Aufeinanderbeißen und/oder eine Temperatur zu erfassen.

13. Verwendung eines Multisensorsystem (100) nach einem der Ansprüche 1 bis 12 zum Erfassen von Messdaten in einer Mundhöhle.

## Claims

1. A multi-sensor system (100) having several dental sensors (101-S; 101-M) for placement in an oral cavity, comprising:
- several slave dental sensors (101-S) for acquiring measurement data in the oral cavity, each comprising a transmitting device (103) for transmitting the measurement data; and
- a master dental sensor (101-M) having a receiving device (109-M) for receiving the measurement data from the individual slave dental sensors (101-S) which is configured to retrieve measurement data successively from the individual slave dental sensors (101-S).

2. The multi-sensor system (100) according to claim 1, wherein the slave dental sensor (101-S) comprises an attachment device (117) or attachment means for attaching the slave dental sensor (101-S) to a tooth and/or the master dental sensor (101-M) comprises an attachment device (117) or attachment means for attaching the master dental sensor (101-M) to a tooth.

3. The multi-sensor system (100) according to any one of the preceding claims, wherein the transmitting device (103) of the slave dental sensor (101-S) comprises a wired and/or a wireless interface (107).

4. The multi-sensor system (100) according to any one of the preceding claims, wherein the slave dental sensor (101-S) comprises a receiving device (109) for receiving data from the master dental sensor (101-M).

5. The multi-sensor system (100) according to any one of the preceding claims, wherein the slave dental sensor (101-S) is configured to be controlled by the master dental sensor (101-M).

6. The multi-sensor system (100) according to any one of the preceding claims, wherein the master dental sensor (101-M) comprises a transmitting device (111) for transmitting the measurement data to a node (115) outside the oral cavity.

7. The multi-sensor system (100) according to any one of the preceding claims, wherein the slave dental sensor (101-S) and/or the master dental sensor (101-M) comprise an energy storage for autonomous operation.

8. The multi-sensor system (100) according to any one of the preceding claims, wherein the master dental sensor (101-M) comprises a microprocessor (115) for processing the measurement data.

9. The multi-sensor system (100) according to any one of the preceding claims, wherein the master dental sensor (101-M) is configured to compress and/or encrypt the measurement data.

10. The multi-sensor system (100) according to any one of the preceding claims, wherein the slave dental sensor (101-S) comprises a digital memory (119) for storing a measurement program (121) and a microprocessor (115) for executing the measurement program (121).

11. The multi-sensor system (100) according to any one of the preceding claims, wherein the multi-sensor system (100) is configured to transmit the measurement data to an external application.

12. The multi-sensor system (100) according to any one of the preceding claims, wherein the slave dental sensor (101-S) is configured to detect a pH, an ethanol concentration, a lactate concentration, a cortisol concentration, a glucose concentration, and/or an ion concentration, or to detect sound waves during biting together and/or a temperature.

13. A use of a multi-sensor system (100) according to any one of claims 1 to 12 for acquiring measurement data in an oral cavity.

## Revendications

1. Système à capteurs multiples (100) comprenant plusieurs capteurs dentaires (101-S ; 101-M) destinés à être placés dans une cavité buccale, comprenant :
- plusieurs capteurs dentaires esclaves (101-S) pour saisir des données de mesure dans la cavité buccale, chacun comprenant un d'émission (103) pour l'émission des données de mesure ; et
- un capteur dentaire maître (101-M) avec un dispositif récepteur (109-M) pour recevoir les données de mesure des capteurs dentaires esclaves individuels (101-S), qui est conçu pour interroger séquentiellement les données de mesure des capteurs dentaires esclaves individuels (101-S).

2. Système à capteurs multiples (100) selon la revendication 1, où le capteur dentaire esclave (101-S) comprend un dispositif de fixation (117) ou des moyens de fixation pour fixer le capteur dentaire esclave (101-S) à une dent et/ou le capteur dentaire maître (101-M) comprend un dispositif de fixation (117) ou des moyens de fixation pour fixer le capteur dentaire maître (101-M) à une dent.

3. Système à capteurs multiples (100) selon l'une des revendications précédentes, où le dispositif d'émission (103) du capteur dentaire esclave (101-S) comprend une interface filaire et/ou une interface sans fil (107).

4. Système à capteurs multiples (100) selon l'une des revendications précédentes, où le capteur dentaire esclave (101-S) comprend un dispositif de réception (109) pour recevoir des données du capteur dentaire maître (101-M).

5. Système à capteurs multiples (100) selon l'une des revendications précédentes, où le capteur dentaire esclave (101-S) est configuré pour être commandé par le capteur dentaire maître (101-M).

6. Système à capteurs multiples (100) selon l'une des revendications précédentes, où le capteur dentaire maître (101-M) comprend un dispositif d'émission (111) pour transmettre les données de mesure à un nœud (115) situé en dehors de la cavité buccale.

7. Système à capteurs multiples (100) selon l'une des revendications précédentes, où le capteur dentaire esclave (101-S) et/ou le capteur dentaire maître (101-M) comprennent une unité de stockage d'énergie pour un fonctionnement autonome.

8. Système à capteurs multiples (100) selon l'une des revendications précédentes, où le capteur dentaire maître (101-M) comprend un microprocesseur (115) pour le traitement des données de mesure.

9. Système à capteurs multiples (100) selon l'une des revendications précédentes, où le capteur dentaire maître (101-M) est conçu pour compresser et/ou crypter les données de mesure.

10. Système à capteurs multiples (100) selon l'une des revendications précédentes, où le capteur dentaire esclave (101-S) comprend une mémoire numérique (119) pour stocker un programme de mesure (121) et un microprocesseur (115) pour exécuter le programme de mesure (121).

11. Système à capteurs multiples (100) selon l'une des revendications précédentes, où le système à capteurs multiples (100) est configuré pour transmettre les données de mesure à une application externe.

12. Système à capteurs multiples (100) selon l'une des revendications précédentes, où le capteur dentaire esclave (101-S) est configuré pour détecter une valeur de pH, une concentration d'éthanol, une concentration de lactate, une concentration de cortisol, une concentration de glucose et/ou une concentration d'ions ou pour détecter des ondes sonores lors du mordillement et/ou une température.

13. Utilisation d'un système à capteurs multiples (100) selon l'une des revendications 1 à 12 pour l'acquisition de données de mesure dans une cavité buccale.
